# EUROPEAN PATENT SPECIFICATION

(11) **EP 2 700 116 B1**
(45) Date of publication and mention of the grant of the patent: **14.11.2018**
(21) Application number: 12721981.4
(22) Date of filing: 18.04.2012
(51) Int. Cl.: H01M 2/10, H02J 9/06, A61M 1/10, A61M 1/12

(54) **MULTI POWER SOURCE POWER SUPPLY**
STROMVERSORGUNG MIT MEHRFACHER STROMQUELLE
ALIMENTATION ÉLECTRIQUE MULTISOURCE

(30) Priority: 18.04.2011 US 201113089128
(43) Date of publication of application: 26.02.2014
(73) Proprietor: Maquet Cardiovascular LLC, Wayne, NJ 07470 (US)
(72) Inventor: KAUSHANSKY, Yefim, North Haledon New Jersey 07508-3308 (US)
(74) Representative: Viering, Jentschura & Partner mbB Patent- und Rechtsanwälte
(86) International application number: PCT/US2012/034116
(87) International publication number: WO 2012/145424

(56) References cited:
- EP-A1- 0 825 520
- EP-A2- 1 030 240
- WO-A2-01/61822
- GB-A- 2 311 408
- US-A- 4 016 871
- US-A- 5 011 468
- US-A- 5 508 569
- US-A1- 2006 211 297
- US-A1- 2010 264 738

## Description

### BACKGROUND OF THE INVENTION

### Field of the Invention

The present invention generally relates to a battery housing. An exemplary embodiment of the present invention relates to a battery housing for a medical device such as an intra-aortic balloon pump.

### Description of Related Art

Intra-aortic balloon pumps (IABPs) are used to provide pneumatic assistance to a failing or weakened heart. Often this therapy must be sustained as patients are transported within or between medical facilities.

When an IABP is in a stationary mode, it is typically powered by the medical facility's AC power. The IABP incorporates an internal back-up battery used to provide power during transport or in case of AC power loss. Typically, the battery is integrated into an internal battery compartment and is not readily accessible for replacement while the IABP is in operation. Therefore, if the battery is allowed to discharge, the IABP becomes unusable for patient transport and must be connected to an AC power source for recharging, some times for several hours.

IABPs typically use lead acid battery technology. This type of battery is heavy, bulky, and requires fasteners to connect to the terminals. Due to the heavy weight of a lead- acid battery, heavy brackets are required to secure it within the IABP. Further, lead-acid batteries have a finite number of charge/discharge cycles after which they are no longer capable of retaining a charge.

Replacement of a battery of this type is also labor intensive. Replacement typically requires a technician to disassemble a portion of the IABP so as to disconnect the battery compartment and remove it from the IABP. The battery compartment must then be partially disassembled before the battery can be removed. The technician then reverses this process to install the new battery. During this time, the IABP is out of service.

Battery technology has made significant advances over the past years. As a result, new batteries weigh less, take less physical space, and have higher power density ratings than their predecessors. A need exists for a medical unit such as an IABP with an improved, safer, and more easily replaceable battery.

US 2011/264738 A1 discloses an intelligent battery system for powering a mobile workstation in which the system includes two hot-swap batteries and a spare battery that are all releasably attached to a respective snap-on interface bracket, and a locking device of one of the snap-on interface brackets that prevents accidental simultaneous removal of the two batteries powering the workstation.

US 5,011,468 A discloses a retroperfusion and retroinfusion control apparatus that includes a console pump for pumping blood and a retroperfusion catheter balloon that is inflated and deflated in synchronism with each pump stroke or in response to sensed sinus pressure. Further, a gas inflation mechanism is provided to inflate and deflate the balloon, which is placed in blood vessels such as the great cardiac vein or the jugular veins. This apparatus includes a power supply for the pump console and a backup power supply.

GB 2 311 408 A discloses a portable apparatus and battery locking mechanism, wherein the portable apparatus includes a battery case portion that includes a first region providing a space for a first battery, a second region providing a space for a second battery, and a third region located between the first region and the second region, wherein the third region is used for loading and releasing only one of the first battery and the second battery at a time. Further, a slide member is disclosed which is located slidably at the third region and having an engaging portion that contacts one of the first battery and the second battery, and a locking member that engages the slide member when one of the first battery and second battery is taken out from its corresponding region of the battery case portion. Under these conditions, the slide member so locked locks the other remaining battery in its corresponding region so current flow is assured and data destruction is prevented.

EP 1 030 240 A2 discloses an electronic price label battery storage apparatus and replacement method. EP 0 825 520 A1 discloses portable information equipment that has two battery holding sections and two battery holders, each containing a battery. Each one of these two documents discloses the use of multiple batteries and a structure for ensuring that at least one battery maintains power to electronics when other batteries are removed.

US 4 016 871 A1 discloses an intra-aortic balloon pump system according to the preamble of claim 1.

### SUMMARY OF THE INVENTION

The present invention provides an intra-aortic balloon pump system and a method for using the power supply system of this intra-aortic balloon pump system as featured in the independent claims. Preferred embodiments of the present invention are described in the dependent claims.

A medical device, such as an intra-aortic balloon pump, according to an example embodiment of the present invention includes a pump, a lock element, a power-source housing, and a control unit adapted to control the pump. An interior of the power-source housing including a first housing part and a second housing part. The first housing part is configured to hold a first power source. The second housing part is configured to hold a second power source. The first housing part includes a first opening large enough for the first power source to pass through. The second housing part includes a second opening large enough for the second power source to pass through. The power-source housing is accessible such that the first power source and the second power source can be replaced without dismantling the intra-aortic balloon pump. The power-source housing is configured such that when the first power source is being removed from the first housing part the lock element prevents simultaneous removal of the second power source from the second housing part and when the second power source is being removed from the second housing part the lock element prevents simultaneous removal of the first power source from the first housing part.

According to an example embodiment, the first power source is capable of being replaced while the second power source is powering the intra-aortic balloon pump and the second power source is capable of being replaced while the first power source is powering the intra-aortic balloon pump.

According to an example embodiment, each of the first and second power sources is independently capable of providing sufficient power to run the intra-aortic balloon pump for a predetermined period of time.

According to an example embodiment, at least one of the first power source and the second power source is a battery.

According to an example embodiment, at least one of the first power source and the second power source is an AC power supply or a DC power supply.

According to an example embodiment, the first power source is a battery and the second power source is an AC power supply or a DC power supply. Further, the first power source and the second power source may have similar physical dimensions.

According to an example embodiment, the lock element includes a mechanism positioned between the first and second openings. The mechanism is movable between (i) a first position at least partially blocking the first opening but not the second opening, (ii) a second position at least partially blocking the first opening and the second opening, and (iii) a third position at least partially blocking the second opening but not the first opening.

According to an example embodiment, the lock element includes a solenoid configured to move a rod forward such that a forward end of the rod passes into a recess in the lock element to fix movement of the lock element.

According to an example embodiment, the power-source housing is configured to facilitate removal of the first power source. When the first housing part is transitioned from a locked state to an unlocked state, via the lock element, the first power source is shifted forward in the power-source housing a predetermined distance by one or more mechanisms in the power-source housing.

According to an example embodiment, the one or more mechanisms in the power-source housing are spring-based.

According to an example embodiment, the power-source housing is configured to facilitate removal of the second power source. When the second housing part is transitioned from a locked state to an unlocked state, via the lock element, the second power source is shifted forward in the power-source housing a predetermined distance by the one or more mechanisms in the power-source housing.

According to an example embodiment, the mechanism includes a first rod. The first rod is configured such that placing the first power source in the first housing part to a sufficient depth within the power-source housing shifts the first rod rearward away from the first opening against the force of a first spring-based element. Subsequent removal of the first battery causes the first rod to move forward such that a forward end of the first rod passes into a recess in the lock element while in the third position.

According to an example embodiment, the mechanism further includes a second rod. The second rod is configured such that placing the second power source in the second housing part to a sufficient depth within the housing shifts the second rod rearward away from the second opening against the force of a second spring-based element. Subsequent removal of the second battery causes the second rod to move forward such that a forward end of the second rod passes into a recess in the lock element while in the first position.

According to an example embodiment, the mechanism further includes a first solenoid and a second solenoid. The lock element contains a first partial recess groove, configured to engage a first rod controlled by the first solenoid, and a second partial recess groove, configured to engage a second rod controlled by the second solenoid. The first partial recess groove when engaged by the first rod both allows motion between the first position and the second position and restricts motion from the second position to the third position. The second partial groove when engaged by the second rod both allows motions between the second position and the third position and restricts motion from the second position to the first position.

A container according to an example embodiment of the present invention includes a housing and a lock element. The housing defines a first housing part and a second housing part. The first housing part is configured to hold a first power source. The second housing part is configured to hold a second power source. The first housing part includes a first opening large enough for the first power source to pass through. The second housing part includes a second opening large enough for the second power source to pass through. The container is configured such that when the first power source is being removed from the first housing part, the lock element prevents simultaneous removal of the second power source from the second housing part and when the second power source is being removed from the second housing part, the lock element prevents simultaneous removal of the first power source from the first housing part.

According to an example embodiment, the container is configured to facilitate removal of the first power source. When the first housing part is transitioned from a locked state to an unlocked state, via the lock element, the first power source is shifted forward in the housing a predetermined distance by one or more mechanisms in the housing.

According to an example embodiment, the container is configured to facilitate removal of the second power source. When the second housing part is transitioned from a locked state to an unlocked state, via the lock element, the second power source is shifted forward in the housing a predetermined distance by the one or more mechanisms in the housing.

According to an example embodiment, the lock element includes a mechanism positioned between the first and second housing parts. The mechanism includes a knob configured to be moved to (i) a first position at least partially blocking the first opening but not the second opening, (ii) a second position at least partially blocking the first opening and the second opening, and (iii) a third position at least partially blocking the second opening but not the first opening.

According to an example embodiment, movement of the knob from the first position to either the second or third position is achieved through rotation of the knob.

A device according to an example embodiment of the present invention includes a power source, a lock element, and a power-source housing. An interior of the power-source housing includes a first housing part and a second housing part. The first housing part is configured to hold a first power source. The second housing part is configured to hold a second power source. The first housing part includes a first opening large enough for the first power source to pass through. The second part includes a second opening large enough for the second power source to pass through. The power-source housing is configured such that when the first power source is being removed from the first housing part the lock element prevents simultaneous removal of the second power source from the second housing part and when the second power source is being removed from the second housing part the lock element prevents simultaneous removal of the first power source from the first housing part.

The present invention is not limited to medical equipment. In an example embodiment, the device is a piece of machinery, such as drill, or an electric car, or a computer, or a mobile phone, or any other device that would benefit from an uninterrupted power supply.

A method, according to an exemplary embodiment of the present invention, involves loading and replacing one more batteries in an intra-aortic balloon pump. The intra-aortic balloon pump including a housing containing at least a first and second battery. The method includes (i) inserting the first battery into a first battery compartment in the housing, (ii) inserting the second battery into a second battery compartment in the housing, (iii) positioning a knob, rotatably connected to the housing, at least partially in front of the second battery but not the first battery, and (iv) removing the first battery, the knob locking the second battery in the second battery compartment until the first battery is replaced and the knob repositioned.

In an exemplary embodiment, the first battery automatically being partially forced out of the first battery compartment as a result of the knob positioning.

In an exemplary embodiment, the second battery is used to fully operate the intra-aortic balloon pump after the first battery is removed from the housing.

A method, according to an example embodiment of the present invention, minimizes or completely avoids power supply disruption to a device. The device, e.g., a medical device such as an intra-aortic balloon pump or a ventilator, etc., includes a housing defining a first housing part and a second housing part and a lock element connected to the housing. The first housing part is configured to hold a first power source and the second housing part is configured to hold a second power source. The first housing part includes a first opening large enough for the first power source to pass through and the second housing part including a second opening large enough for the second power source to pass through. The method includes the steps of: (a) while the first power source is in the first housing part and the second power source is in the second housing part, moving the lock element from a first position at least partially blocking both the first opening and the second opening to a second position at least partially blocking the first opening and not blocking the second opening, such that the second power source can be removed from the second housing part and the first power source cannot be simultaneously removed from the first housing part; and (b) removing the second power source from the second housing part.

According to an example embodiment, the lock element is positioned between the first and second openings and moveable between (i) a first position, at least partially blocking the first opening but not the second opening, (ii) a second position at least partially blocking the first opening and the second opening, and (iii) a third position at least partially blocking the second opening but not the first opening.

According to an example embodiment, the lock element is a latch-type mechanism including a latch. In the first position, the latch blocks removal of both the first and second power sources. In the second position, the latch blocks removal of only the first power source. In the third position, the latch block removal of only the second power source.

According to an example embodiment, the method further includes the step of replacing the second power source and then moving the lock element back to the first position.

According to an example embodiment, the method further includes the step of restricting motion of the lock element away from the first position until the second power source has been replaced.

A power supply system, according to an exemplary embodiment of the present invention, includes a first housing part, configured to removably receive a first power source through a first opening of the first housing part, and a second housing part, configured to removably receive a second power source through a second opening of the second housing part. The power supply system further includes a first electrical connector for removably connecting the first power source to a device and a second electrical connector for removably connecting the second power source to the device. A lock element of the system is movably positioned with respect to the first and second housing parts to prevent simultaneous removal of the first and second power sources from the first and second housing parts, wherein the lock element is configured to secure the first power source within the first housing part if the second power source is removed from the second housing part.

According to an exemplary embodiment, the lock element is configured to secure the second power source within the second housing part if the first power source is removed from the first housing part.

According to an exemplary embodiment, the lock element is configured to require at least one of the first and second power sources be locked within one of the first and second housing parts and electrically connected to one of the first and second electrical connectors at all times.

According to an exemplary embodiment, the lock element includes a knob movably positioned relative to the first and second housing parts to secure the first power source in electrical connection with the first electrical connector if the second power source is removed from the second housing part and to secure the second power source in electrical connection with the second electrical connector if the first power source is removed from the first housing part.

According to an exemplary embodiment, the lock element is selected from a sliding latch, a sliding bolt latch and a sliding bolt locking latch.

According to an exemplary embodiment, the lock element includes a knob and wherein a groove formed in the knob cooperates with a first projection member of the lock element to restrict movement of the knob.

According to an exemplary embodiment, the lock element includes a solenoid configured to move a rod such that an end of the rod is received within a recess of the lock element fixing the position of the lock element, so as to secure the first power source within the first housing part if the second power source is removed from the second housing part and so as to secure the second power source within the second housing part if the first power source is removed from the first housing part.

According to an exemplary embodiment, the lock element is movably positioned between the first and second openings, such that the lock element is movable between a first position at least partially blocking the first opening while permitting unobstructed access to the second opening and at least one of: a second position wherein the lock element at least partially blocks the first opening and the second opening and a third position wherein the lock element at least partially blocks the second opening while permitting unobstructed access to the first opening.

According to an exemplary embodiment, the lock element includes a first solenoid and a second solenoid. The lock element further includes a first groove configured to engage a first rod controlled by the first solenoid and a second groove configured to engage a second rod controlled by the second solenoid. The first groove when engaged by the first rod allows the lock element to be moved between the first position and the second position and restricts movement from the second position to the third position. The second groove when engaged by the second rod allows the lock element to move between the second position and the third position and restricts movement from the second position to the first position.

According to an exemplary embodiment, the power supply system includes an ejection mechanism including a first spring-based element to facilitate removal of the first power source from the first housing part.

According to an exemplary embodiment, the ejection mechanism includes an electro-mechanical servo and switch system to direct the positioning of the lock element based on a condition of one of the first and second power sources detected by the electro-mechanical servo.

According to an exemplary embodiment, the ejection mechanism includes a first rod and a second projection member, positioned on the first rod, for engaging the first power source. The first rod is attached to a second spring-based element and is operatively associated with the lock element. Upon insertion of the first power source within the first housing part, the first power source bears against the second projection member moving the first rod rearward away from the first opening and compressing the second spring-based element. Upon removing the first power source from the first housing part the second spring-based element expands applying a forward force to the first rod and the second projection member pushing the first power source towards the first opening.

According to an exemplary embodiment, the ejection mechanism includes a third spring-based element, a second rod and a third projection member, positioned on the second rod, for engaging the second power source, The second rod is attached to the third spring-based element and is operatively associated with the lock element. Upon insertion of the second power source within the second housing part, the second power source bears against the third projection member moving the second rod rearward away from the second opening and compressing the third spring-based element. Upon removing the second power source from the second housing part the third spring-based element expands applying a forward force to the second rod and the third projection member pushing the second power source towards the second opening.

According to an exemplary embodiment, the ejection mechanism is positioned between the first and second housing parts containing the first and second power sources.

According to an exemplary embodiment, upon removing the first power source from the first housing part an end of the first rod is received within one or more recesses of the lock element, fixedly positioning the lock element so as to at least partially block a portion of the second opening. Upon removing the second power source from the second housing part, an end of the second rod is received within the one or more recesses of the lock element fixedly positioning the lock element so as to at least partially block a portion of the first opening.

According to an exemplary embodiment, the first and second housing parts form a unitary chamber defining the power supply housing.

According to an exemplary embodiment, the first and second housing parts are separated by a divider. The first housing part and the divider define a first receptacle sized for containing the first power source. The second housing part and the divider define a second receptacle for sized for containing the second power source.

According to an exemplary embodiment, the power supply system includes first and second power sources. The first and second power sources may be one or more batteries, one or more AC power supply modules or one or more DC power supply modules.

According to an exemplary embodiment, the first and second power sources can be batteries. The first power source can be a battery while the second power source is an AC or DC power supply module. Both the first and second power sources can also be AC or DC power supply modules.

A system, according to an exemplary embodiment of the present invention, includes a device and a power supply system electrically connected to and supplying power to the device. The power supply system includes a first housing part configured to removably receive a first power source through a first opening of the first housing part, wherein the first housing part including a first electrical connector for electrically connecting the first power source to the device. A second housing part of the power supply system is configured to removably receiving a second power source through a second opening of the second housing part, wherein the second housing part includes a second electrical connector for electrically connecting the second power source to the device. A lock element of the power supply system is movably positioned with respect to the first and second housing parts to prevent simultaneous removal of the first and second power sources from the first and second housing parts. The lock element is configured to secure the first power source within the first housing part if the second power source is removed from the second housing part, and the lock element is configured to secure the second power source within the second housing part if the first power source is removed from the first housing part.

According to an exemplary embodiment, the system is a portable medical device system.

According to an exemplary embodiment, the system includes a control unit operatively associated with the power supply system, wherein the control unit includes a processor.

According to an exemplary embodiment, the lock element is configured to be oriented in a fixed position relative to the first and second housing parts until one or more conditions of the first and second power sources detected by the control unit are satisfied.

According to an exemplary embodiment, the system includes a bus and a user interface display, each operatively associated with the control unit.

According to an exemplary embodiment, the system is an intra-aortic balloon pump system, and the device electrically connected to the power supply system is a pump for inflating and deflating a balloon of an intra-aortic balloon catheter.

According to an exemplary embodiment, the system may include any of the above described embodiments of the power supply system to supply power to the device.

A power supply system, according to an exemplary embodiment of the present invention, includes a first housing part configured to removably receive a first power source through a first opening of the first housing part and a second housing part configured to removably receive a second power source through a second opening of the second housing part, wherein the first and second housing parts form a power supply housing. The power supply system further includes a first electrical connector for removably connecting the first power source to a device and a second electrical connector for removably connecting the second power source to the device. The power supply housing is configured to require at least one of the first and second power sources be locked within one of the first and second housing parts at all times in an electrically connected position for delivering power to a device, wherein the housing is configured to lock the first power source within the first housing part if the second power source is removed from the second housing part and wherein the power supply housing is configured to lock the second power source within the second housing part if the first power source is removed from the first housing part.

A power supply system, according to an exemplary embodiment of the present invention, includes a first housing part configured to removably receive a first power source through a first opening of the first housing part and a second housing part configured to removably receive a second power source through a second opening of the second housing part, wherein the first and second housing parts form a power supply housing. The power supply system further includes a first electrical connector for removably connecting the first power source to a device and a second electrical connector for removably connecting the second power source to the device. The power supply housing is configured to prevent simultaneous removal of the first and second power sources from the first and second housing parts, wherein the housing is configured to secure the first power source within the first housing part if the second power source is removed from the second housing part and wherein the housing is configured to secure the second power source within the second housing part if the first power source is removed from the first housing part. A housing component can be configured and movably positioned relative to the first and second housing parts to prevent simultaneous removal of the first and second power sources from the first and second housing parts, wherein the housing component is configured to secure the first power source within the first housing part if the second power source is removed from the second housing part and wherein the housing component is configured to secure the second power source within the second housing part if the first power source is removed from the first housing part.

A power supply system, according to an exemplary embodiment of the present invention, includes a first housing part configured to removably receive a first power source through a first opening of the first housing part and a second housing part configured to removably receive a second power source through a second opening of the second housing part. The power supply system further includes a first electrical connector that electrically connects the first power source to a device and a second electrical connector that electrically connects the second power source to the device. A lock element of the power supply system is configured and movably positioned with respect to the first and second housing parts to require at least one of the first and second power sources is locked within one of the first and second housing parts and connected to one of the first and second electrical connectors at all times. The lock element secures the first power source in electrical connection with the first electrical connector if the second power source is removed from the second housing part, and the lock element secures the second power source in electrical connection with the second electrical connector if the first power source is removed from the first housing part.

A power supply system, according to an exemplary embodiment of the present invention, includes a first housing part configured to removably receive a first power source through a first opening of the first housing part and a second housing part configured to removably receive a second power source through a second opening of the second housing part. The power supply system further including a first electrical connector for removably connecting the first power source to a device and a second electrical connector for removably connecting the second power source to the device. A lock element of the power supply system is movably positioned with respect to the first and second housing parts to require at least one of the first and second power sources is locked within one of the first and second housing parts at all times. The lock element can be oriented in: a first position securing the first power source within the first housing part if the second power source is removed from the second housing part; a second position securing the second power source within the second housing part if the first power source is removed from the first housing part; and a third position securing the first power source within the first housing part and simultaneously securing the second power source within the second housing part.

A system, according to an exemplary embodiment of the present invention, includes a device as well as a first and second power source for supplying power to the device. A first housing part of the system is configured to removably receive a first power source through a first opening of the first housing part, wherein the first housing part includes a first electrical connector for electrically connecting the first power source to the device. A second housing part of the system is configured to removably receive a second power source through a second opening of the second housing part, wherein the second housing part comprises a second electrical connector for electrically connecting the second power source to the device. A lock element of the system is configured and movably positioned with respect to the first and second housing parts to require at least one of the first and second power sources is locked within one of the first and second housing parts and connected to one of the first and second electrical connectors at all times. The lock element secures the first power source in electrical connection with the first electrical connector if the second power source is removed from the second housing part, and the lock element secures the second power source in electrical connection with the second electrical connector if the first power source is removed from the first housing part.

An intra-aortic balloon pump system, according to an exemplary embodiment of the present invention, includes a pump for inflating and deflating a balloon of an intra-aortic balloon catheter, a first power source for supplying power to the pump, a second power source for supplying power to the pump, and a power supply system. The power supply system includes a first housing part configured to removably receive the first power source through a first opening of the first housing part and a second housing part configured to removably receiving the second power source through a second opening of the second housing part. A lock element of the power supply system is movably positioned with respect to the first and second housing parts to prevent simultaneous removal of the first and second power sources from the first and second housing parts. The lock element is configured to secure the first power source within the first housing part if the second power source is removed from the second housing part and is also configured to secure the second power source within the second housing part if the first power source is removed from the first housing part.

A method for using a power supply system, according to an exemplary embodiment of the present invention, involves the use of a power supply system including a first housing part capable of removably receiving the first power source through a first opening of the first housing part, a second housing part capable of removably receiving the second power source through a second opening of the second housing part and a lock element. The method involves the steps of movably positioning the lock element relative to the first and second housing parts to secure the first power source within the first housing part if the second power source is removed from the second housing part and movably positioning the lock element relative to the first and second housing parts to secure the second power source within the second housing part if the first power source is removed from the first housing part, so as to prevent simultaneous removal of the first and second power sources from the first and second housing parts.

According to an exemplary embodiment, the method involves movably positioning the lock element relative to the first and second housing parts to secure the first power source within the first housing part and simultaneously securing the second power source within the second housing part.

According to an exemplary embodiment, the method involves moving the lock element to a first position at least partially blocking the first opening when the first power source is contained within the first housing part while permitting unobstructed access to the second opening and removing the second power source from the second housing part.

According to an exemplary embodiment, the method involves preventing movement of the lock element away from the first position until the second power source is replaced within the second housing part.

According to an exemplary embodiment, the method involves inserting the second power source within the second housing part and moving the lock element to a second position so as to at least partially block the first and second openings, wherein first and second power sources are secured within the first and second housing parts.

According to an exemplary embodiment, the method involves moving the lock element to a third position at least partially blocking the second opening while permitting unobstructed access to the first opening and removing the first power source from the first housing part.

According to an exemplary embodiment, the first and second power sources of the power supply system are operatively associated with a control unit, and the method involves selectively accessing the lock element based upon a condition of the first and second power sources detected by the control unit.

According to an exemplary embodiment, the condition detected by the control unit is selected from the group consisting of: a voltage, a current, a temperature, an expected battery life and combination thereof.

According to an exemplary embodiment, the method involves hot-swapping the first and second power sources.

According to an exemplary embodiment, the method is performed using any of the above described embodiments of the power supply systems.

According to an exemplary embodiment, the method is performed using the any one of the above described systems. In particular, the method may be performed with a portable medical device system, such as an intra-aortic balloon pump system.

Reference throughout this specification to "an embodiment" or "an example embodiment" or "an exemplary embodiment" means that a particular feature, structure, or characteristic described in connection with the embodiment is included in at least one embodiment of the present invention. Thus, the appearances of these phrases in various places throughout this specification are not necessarily all referring to the same embodiment. Furthermore, the particular features, structures, or characteristics may be combined in any suitable manner in one or more embodiments.

An example embodiment of the present invention is described in more detail below with reference to the appended Figures. The foregoing description and examples have been set forth as mere illustrations and are not intended to be limiting. Each of the disclosed aspects and embodiments may be considered individually or in combination with other aspects, embodiments, and variations thereof. The steps of the methods described herein are not confined to any particular order of performance.

### BRIEF DESCRIPTON OF THE DRAWINGS

Figure 1 is a block diagram illustrating components of an intra-aortic balloon pump system according to an example embodiment of the present invention.
Figures 2A-2D are front views of an IABP power-source housing according to an example embodiment. Figures 2A-2C illustrate different positions of a lock element. Figure 2D is the power-source housing of Figure 2B with one of the power sources ejected.
Figure 3 is a flowchart of a method according to an example embodiment of the present invention.
Figure 4A is a side view of the lock element of Figures 2A-2D.
Figure 4B is a rear view of the lock element of Figures 2A-2D.
Figure 5 is a front perspective view of an example power-source housing with one of the power sources partially ejected.
Figure 6 is a front perspective view of a mechanism from inside the power-source housing of Figure 5 with the knob removed.
Figure 7 is a front perspective view of the power-source housing of Figure 5 with a portion of the case and one of the power-sources not shown.
Figure 8 is a rear perspective view of the power-source housing of Figure 5.
Figure 9 is a flowchart of a method according to an example embodiment of the present invention.
Figure 10 is a rear view of a lock element according to an example embodiment of the present invention.
Figure 11 is a flowchart of a method according to an example embodiment of the present invention.
Figure 12 is an exemplary embodiment of a portable intra-aortic balloon pump system using the power supply system of the present invention.

### DETAILED DESCRIPTION OF THE PRESENT INVENTION

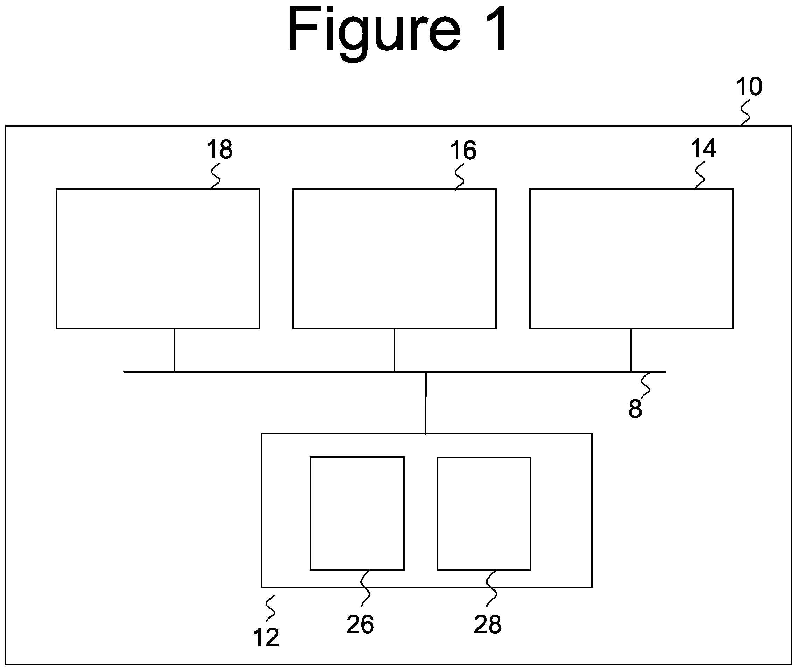

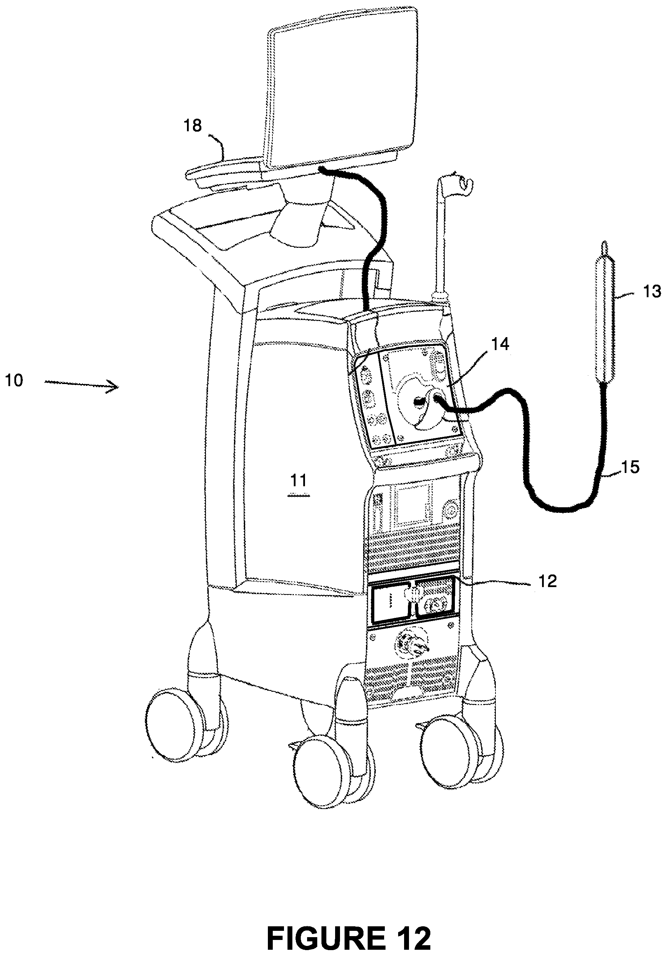

Figure 1 illustrates a block diagram of an intra-aortic balloon pump (IABP) 10 according to an exemplary embodiment of the present invention. The IABP 10 is used to inflate and deflate a balloon 13 on a distal end of a balloon catheter 15, shown in Figure 12. The balloon catheter is typically inserted into a blood vessel of a patient and used to support the patient's heart. As detailed in U.S. Patent No. 6,241,706, herein incorporated by reference in its entirety, the IABP 10 includes a power supply system 12, a pump 14 (used interchangeably herein with compressor), a control unit 16, and bus 8. In the exemplary embodiment shown in Figure 12, the IABP 10 is configured as a portable medical device including a cart 11 with various compartments for holding the power supply system 12, pump 14, control unit 16 and a user interface and display 18. The dual power supply system enables IABP 10 to be operated as a mobile cart-based system or as a standalone system. The power supply system 12 will be discussed in more detail below. The control unit 16 includes a processor/CPU and a computer-readable storage medium for processing and storing data and/or instructions used by any of the other components of the IABP 10. The control unit 16 is operatively associated with the various components of the IABP 10 and communicates with these components through bus 8. The IABP 10 also includes a user input interface and display 18. The user input interface and display 18 is preferably a touch-screen monitor, but can be separated into its functional components of a monitor and a keyboard or a mouse.

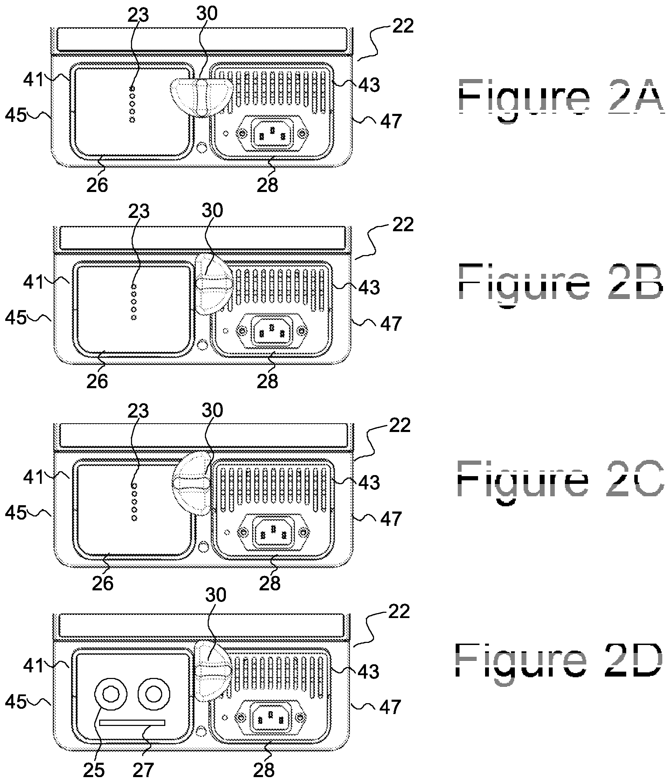

The power supply system 12 of IABP 10 is designed supply power to and minimize the likelihood of power flow disruptions to the IABP 10 by limiting the user's ability to remove, e.g., simultaneously, more than one power source at a time. The power supply system 12 includes multiple user-removable power sources 26, 28 stored in a power-source housing 22. The user-removable power source may be a rechargeable battery, as shown positioned in a left side of power-source housing 22 in Figures 2A-2C. Additionally, the user-removable power source may be an AC and/or DC power-supply module, shown in Figures 2A-2D fitting inside a right side of the power-source housing 22. The AC and/or DC power-supply module is attachable to a power cord that can be plugged into a wall or vehicle power outlet. Alternatively, such a power-supply module may be non-removable.

The power-source housing 22 includes a first housing part 45 and a second housing part 47 for holding and connecting to two user-removable power sources 26, 28, although the housing 22 may be expanded to accommodate additional power sources. The first and second power sources 26, 28 connect to first and second electrical connectors 27 located in the back of the power-source housing 22, as depicted in Fig. 2D. Electrical connectors 27 (the second electrical connector 27 is hidden behind second power source 28) removably connects each of the first and second power sources 26, 28 to a power slot interface and includes 4 power pins and 12 signal pins. The signal pins are used to transmit signals and data through bus 8 to the control unit 16 or various sensors.

In various embodiments, the types of power sources include, or are combinations of, a battery (e.g., a 12-volt rechargeable battery), an AC power supply, a DC power supply, a fuel cell, a photovoltaic cell or other equivalents capable of outputting electrical energy. For example, a battery can be used together with an AC power supply.

In an example embodiment, the IABP 10 can fully operate on the power delivered by one of the first and second power sources 26, 28. Further, when the power of one of the first and second power sources 26, 28 is not available in a sufficient capacity, the IABP 10 may automatically draw power from the remaining power source.

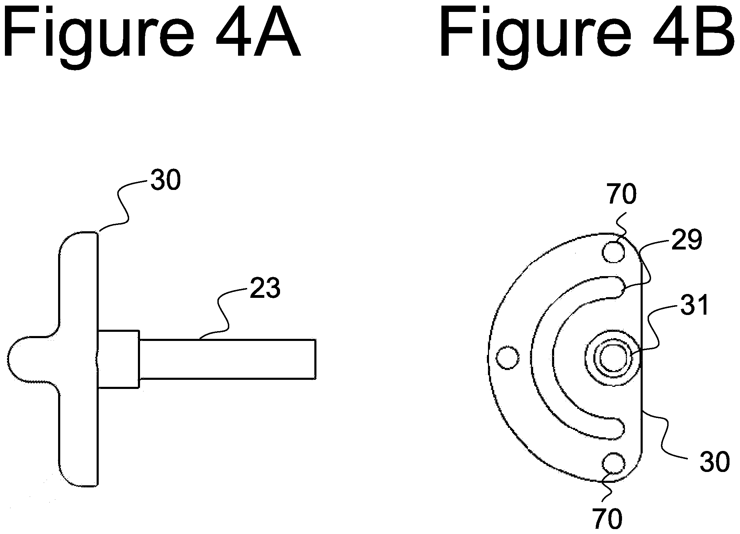

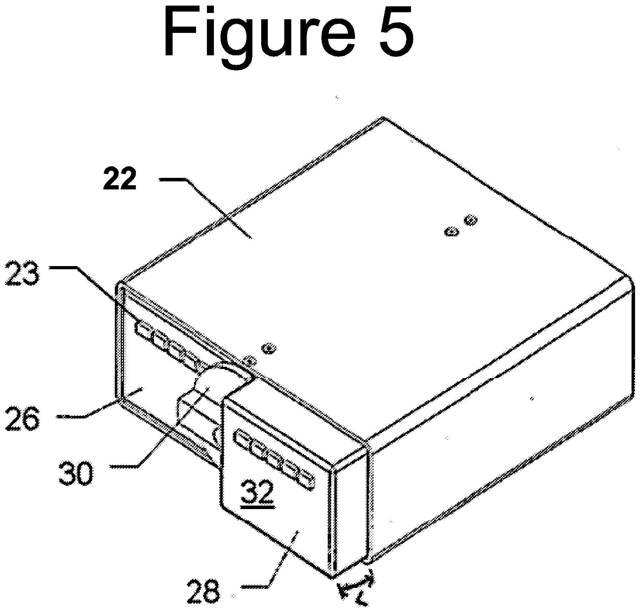

Figures 2A-2D illustrate power-source housing 22 configured to hold two power sources. In this embodiment, power-source housing 22 includes a first and second housing parts 45, 47 forming a unitary chamber for securing multiple power sources 26, 28. As can be seen in the front views of Figures 2A-2C, power-source housing 22 holds a first power source 26, namely a battery, in a receptacle of the first housing part 45 and a second power source 28, namely an AC power-supply module, in a receptacle of the second housing part 47. The receptacle and openings 41, 43 defined by the first and second housing parts 45, 47 are sized to contain first and second power sources 26, 28. A front opening 41 in the first housing part is large enough for a power source 26, such as a battery, pass through and a front opening 43 in the second housing part is large enough for a second power source 28, such as an AC power-supply module, to pass through. The first and second power sources 26, 28 are restrained within housing 22 via a lock element, such as knob 30 as shown in Figures 2A-2C, operatively associated with both the first and second housing parts 45, 47. A lock element, such as knob 30, is hand operated and may be movably positioned by a user to achieve different states of orientation relative to the first and second housing parts 45, 47 to secure the first and second power sources 26, 28 within housing 22. Its motion may be confined to specific positions, for example, as defined by a spring-ball or detent system (shown in Figures 4B and 5). Figure 2D illustrates the power-source housing 22 with first power source 26 removed through the front opening 41.

Power-source housing 22 is configured to prevent the simultaneous removal of the first and second power sources 26, 28 from respective first and second housing parts 45, 47. The housing may be configured to secure first power source 26 within a receptacle of the first housing part 45 in electrical connection with a first electrical connector 27 if the second power source 28 is removed from second housing part 47. The housing may also be configured to secure second power source 28 in a receptacle defined in the second housing part 47 in electrical connection with a second electrical connector 27 if first power source 26 is removed from the first housing part 45. In one embodiment, the ability of housing 22 to secure first and second power sources 26, 28 within housing 22 is achieved by the locking element, which is movably positioned relative to the first and second housing parts 45, 47 to retain at least one of the first and second power sources 26, 28 within first and second housing parts 45, 47 and induce and secure the first and second power sources 26, 28 in electrical connection with the electrical connectors 27.

The power-source housing 22 of the present invention is configured to enable hot-swapping of the first and second power sources 26, 28 so as to avoid a loss of power to the IABP 10. In Figure 2A, first and second power sources 26, 28, which are depicted as a battery and an AC power-supply module, are maintained within the power-source housing 22 by the lock element, knob 30. It is appreciated by a skilled artisan that other power sources and combinations of different power sources, as previously discussed, may also be used in this embodiment. The lock element can be movably positioned to at least partially obstruct or protrude into a portion of one and/or both of the receptacles, specifically the front openings 41, 43, to restrain first and second power sources 26, 28 within housing 22. For example, the edges of knob 30 at least partially block the front openings 41, 43 in the power-source housing 22 such that first and second power sources 26, 28 cannot fit through the partially blocked front openings 41, 43. The power-source housing 22 is designed such that rotating the knob 30 ninety degrees in a counterclockwise direction from its position of Figure 2A to its position in Figure 2B, clears access to and allows for removal of first power source 26 through front opening 41. Rotation of the knob 30 ninety degrees in a clockwise direction from its position of Figure 2A to its position in Figure 2C has the same effect with respect to second power source 28. This configuration prevents simultaneous removal of the first and second power sources 26, 28 from first and second housing parts 45, 47.

The lock element may be configured to be fixed in a specific position. For example, knob 30 may be locked in a third position blocking removal of power source 28 as illustrated in Figures 2B and 2D, until one or more predetermined conditions are met. One condition may be that first power source 26 is reinserted into the first housing part 45. Another condition may be that first power source 26 is charged above a predetermined level, e.g., a level higher than the current charge level of second power source 28 if a battery, as determined by the control unit 16.

Insertion of first and second power sources 26, 28 is completed as follows. In one embodiment, the lock element, specifically knob 30, is rotated to a position allowing unobstructed access to one side of the housing 22. One of first and second power sources 26, 28 is then fully inserted into this side of the housing 22. Next, knob 30 is rotated in the opposite direction, e.g., counter clockwise, over the inserted power sources, providing unobstructed access to the opposite side of the housing 22. The second of power sources 26, 28 is then fully inserted into this opposite side of housing 22. Knob 30 is then rotated back, e.g., clockwise, so that it partially covers the entrance for both of the power sources 26, 28. In this state, both power sources 26, 28 are fully inserted and secured in place via knob 30.

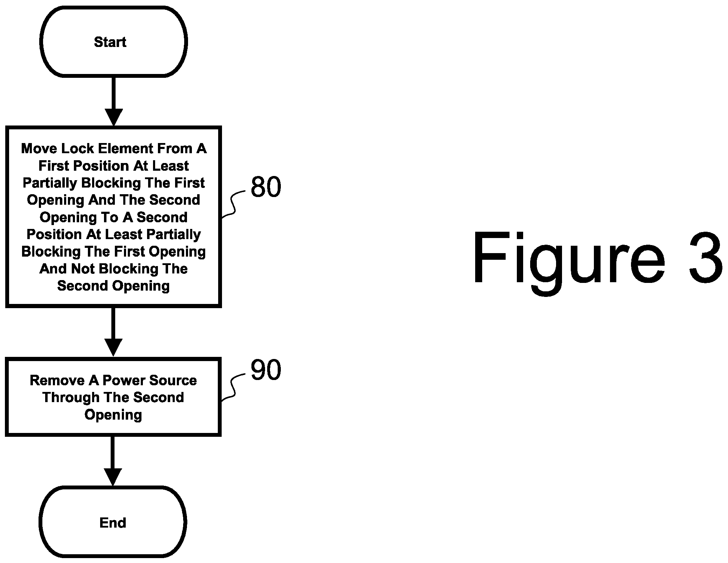

In a similar manner, the first and second power sources 26, 28 can be removed based on providing unobstructed access to the appropriate side of the housing 22. Figure 3 depicts an exemplary embodiment of a method for removing one of a first and second power source 26, 28. In Step 80, a user moves lock element from a first position at least partially blocking the left side of housing 22 and the right side of housing 22 to a second position at least partially blocking the left side of housing 22 and not blocking the right side of housing 22. Then, in Step 90, the user removes one of a first and second power source 26, 28 through the right side of housing 22, which has been unobstructed by Step 80.

The lock element may be implemented in different configurations. For example, the lock element can also be a sliding latch, sliding bolt latch or a sliding bolt locking latch such that the lock element can be confined and optionally locked to specific positions similar to those above. Such a lock can (i) slide to the far left such that only second power source 28 can be removed, (ii) slide to the far right such that only first power source 26 can be removed, or (iii) be centered such that neither of first and second power sources 26, 28 can be removed.

In various embodiments, the IABP 10 can inform the user, e.g., via the user input interface and display 18 or on the battery itself, about which power source can be removed. This benefits the user to ensure that a power source without further power (e.g., an empty battery or an unplugged AC power-supply module) is not the only remaining power source in the system. In one embodiment, the batteries have indicator lights 23 showing how much power is remaining in each battery. In a two-battery system, a user can read the indicators lights to identify which power source is discharged and turn the knob 30 to remove the identified power source. In other embodiments, a sensor or a circuit (not shown) for detecting a power source's characteristics, such as its voltage, can be used to notify the user by displaying a warning or instructions on user input interface and display 18 and/or sounding an audible alarm from the IABP 10, or fix or restrict movement of knob 30 as discussed below.

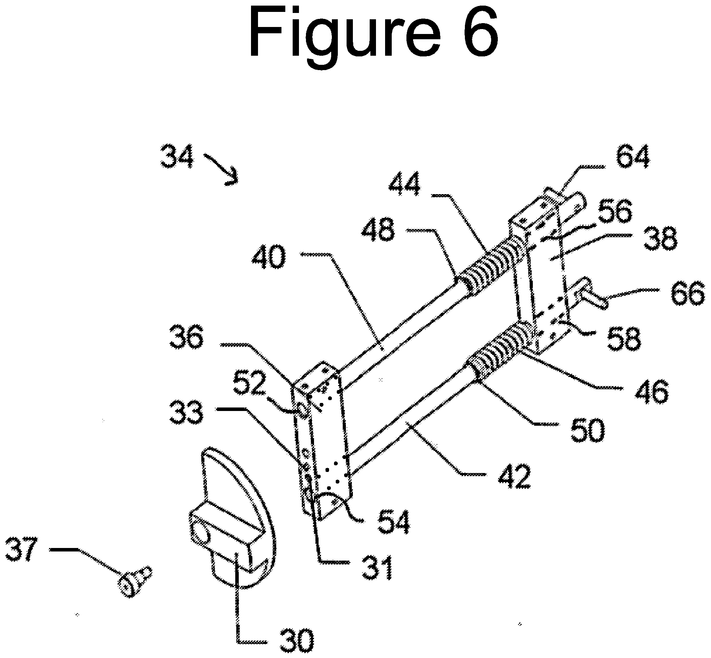

Figures 4A and 4B illustrate a side and rear view of knob 30. The knob 30 is connected to or through the front of power-source housing 22 with an integral connector 23 or a retainer 37 (shown in Figure 6). Knob 30 snaps into place via a detent 31 (shown in Figure 6) or a spring-ball mechanism (not shown) matching up with recess holes 70. A recess groove 29 in a back surface of the knob may cooperate with a first projection member 33 (shown in Figure 6) on the front support 38 (shown in Figure 6) so as to limit the direction of rotation of the knob 30 between the positions shown in Figures 2A-2C. Recess holes 70 can also be used for fixing movement of knob 30 (discussed in more detail later).

The rear of power-source housing 22 may optionally contain internal spring-based elements, as illustrated in Figures 2D (first spring-based elements 25) and Figure 6 (second and third spring-based elements 44, 46), to help eject the first and second power sources 26, 28 from the power source housing 22 as soon as they are unlocked or disengaged. In such embodiments, the power-source housing 22 is designed such that rotating the knob 30 ninety degrees to unblock the partially blocked openings results in the unblocked power source 26, 28 being partially forced out in the forward direction of housing 22 by spring-based elements a length L (see Figure 5) sufficient to allow a user to grasp one the at least partially ejected power source 26, 28 for removal purposes. Length L is preferably less than half of the length of the power source 26, 28, and more preferably, less than a quarter of the length of the power source 26, 28 such that the power source 26, 28 is not completely forced out of housing 22. For example, if a power source is six inches (15.24 cm.) long, then an exemplary length L can be one inch (2.54 cm.). Spring-based elements should have a sufficient spring force to force power sources 26, 28 forward in the case a predetermined distance (length L) while allowing a normal user to insert power sources 26, 28 against the spring force.

Figure 5 shows an exemplary embodiment of a power-source housing 22 having first and second power sources 26, 28 configured as batteries, wherein the second power source 28 is partially forced out in the forward direction a length L due to the exposing of a front surface 32 of the battery.

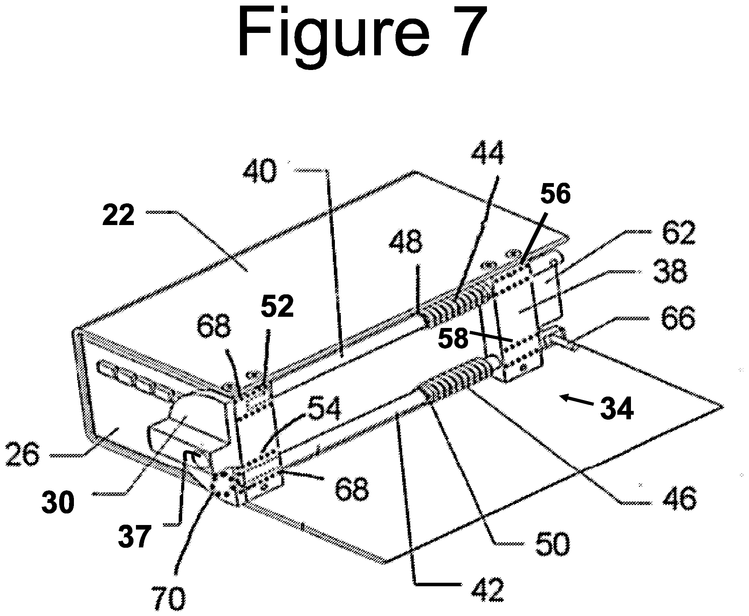

Figure 6 is a perspective view of an ejection mechanism 34 used together with the knob 30 and housing 22 to achieve the above ejection and withdrawal safety features. In one embodiment, ejection mechanism 34 is surrounded on both sides by two sidewalls 60, 62 on either side (shown in Figures 7-8), which form a divider separating the unitary chamber formed by first and second housing parts 45, 47. A receptacle sized for receiving and containing first power source 26 is defined by the divider and first housing part 45. A receptacle sized for receiving and containing second power source 28 is defined by the divider and second housing part 47. Figure 7 shows the housing 22 with the portion over second power source 28 and sidewall 62 removed exposing ejection mechanism 34. The ejection mechanism 34 is positioned in the housing 22 between the first and second power sources 26, 28 and includes front and rear supports 36, 38, first and second rods 42, 40, and second and third spring-based elements 44, 46. Second and third spring-based elements 44, 46 are disposed over and connected to first and second rods 42, 40 via clips 48, 50. First and second rods 42, 40 pass through bores 52, 54 in the front support 36 and bores 56, 58 in the rear support 38 (all four bores shown as dashed lines in Figures 6 and 7). Knob 30 is pivotally secured to front support 36 via a retainer 37.

When knob 30 is rotated such that it extends in front of first power source 26 and no longer restrains forward movement of second power source 28, as illustrated in Figure 5, spring-based element 46 forces first rod 42 forward. Second power source 28 is forced forward by second projection member 66 on first rod 42 a sufficient distance so as to allow a user to grasp the second power source 28 for removal. The forward movement of first rod 42 shifts a forward end 68 of second rod 42 into a recess hole 70 (shown in dashed lines) in a back surface of the knob 30, preventing further rotation of knob 30. Thus, so long as second power source 28 is not replaced or inserted to a sufficient depth to engage second projection member 66, first rod 42 locks knob 30 into position in front of first power source 26 preventing removal of first power source 26. Figure 7 depicts the housing 22 with second power source 28 removed and the portion of the housing 22 surrounding the second power source 28 removed except for the base.

The ejection mechanism 34 works the same way when first power source 26 is removed preventing removal of second power source 28 until first power source 26 is replaced. When knob 30 is rotated such that it extends solely in front of second power source 28, first power source 26 is forced forward by spring-based element 44 (which shifts third projection member 64 on second rod 40) and a forward end 68 of second rod 40 shifts into recess 70 and locks knob 30 in place over second power source 28.

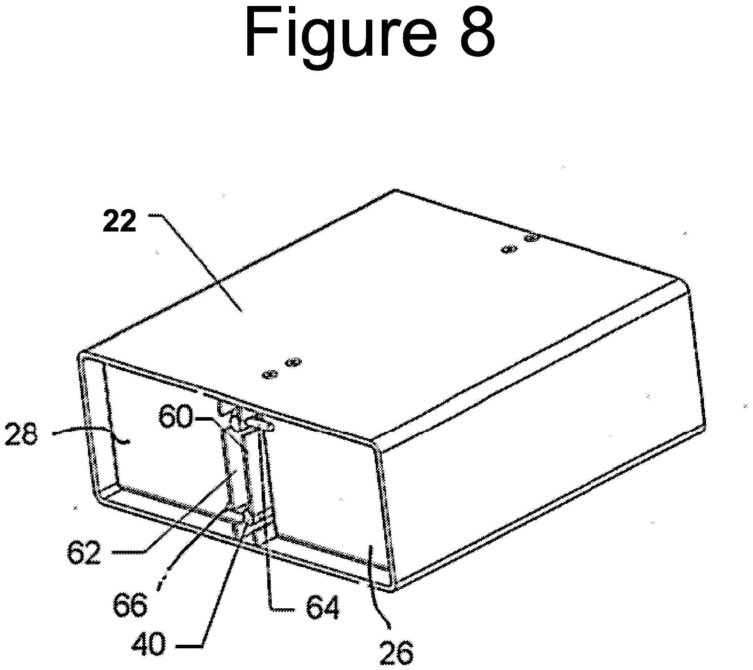

Once first and second power sources 26, 28 are installed in housing 22, only one of the power sources 26, 28 can be removed from the housing 22 at a time. As first power source 26 is positioned in housing 22 it bears against third projection member 64 and forces second rod 40 rearward compressing spring-based element 44. Similarly, as second power source 28 is inserted to a sufficient depth in housing 22, it bears against second projection member 66 forcing first rod 42 rearward compressing spring-based element 46. In this exemplary state, illustrated in Figures 2A and 5, only detente 31 maintains knob 30 in position in front of both first and second power sources 26, 28 configured as batteries. As can be seen in Figure 8, which shows a rear perspective view of housing 22, second and third projection members 64 and 66 lie behind and contact a rear surface of the first and second power sources 26, 28.

In an example embodiment, the spring force of spring-based elements 44, 46 should be sufficient to force first and second power sources 26, 28 forward in housing 22 a predetermined distance, e.g., sufficient for a user to grasp one end of the power source for removal purposes. However, additional spring-based elements connected to the back of first and second power sources 26, 28 or placed within housing 22 may also be used. Alternatively, weaker spring-based elements 44, 46 may be used solely for locking purposes. The first and second power sources 26, 28 may be manually removed, for example, by pulling on a ring or handle (not shown) connected to a front surface of the first and second power sources 26, 28, or partially ejected through spring-based elements 25 (as shown in Figure 2D).

In an example embodiment, the power-source housing 22 is designed so as to assure that at all times one of the first and second power sources 26, 28 remains inside the case in a functioning position, i.e. , a position in which it can deliver current to the IABP 10 through electrical connector 27 in the power-source housing 22. Power-source housing 22 is configured to secure first power source 26 in electrical connection with a first electrical connector 27 if the second power source 28 is removed from second housing part 47. Power-source housing 22 is also configured to secure second power source 28 in electrical connection with a second electrical connector 27 if first power source 26 is removed from the first housing part 45. In one embodiment, the locking capability of housing 22 is achieved by a locking element movably positioned relative to the first and second housing parts 45, 47 to retain at least one of first and second power sources 26, 28 within first and second housing parts 45, 47 and induce and secure electrical connection between the retained first and second power source 26, 28 and the electrical connectors 27 at all times. The lock element therefore positions first and second power sources 26, 28 within the first and second housing parts 45, 47 in an electrically operational position for delivering power to a device. As depicted in Figure 5, when second power source 28 is released from housing 22, first power source 26 is locked in housing 22 by knob 30. Knob 30 may be locked into position over first power source 26 until second power source 28 is fully replaced or alternatively may be rotatable so as not to lock the knob but just to pose an additional obstacle and prevent simultaneous removal of the first and second power sources 26, 28. Similarly, when first power source 26 is released, access to second power source 28 is blocked by knob 30. In the embodiment incorporating a lockable knob 30, knob 30 remains over second power source 28 and is not rotatable until first power source 26 is fully replaced.

In an example embodiment, additional power sources may used to power the IABP 10. For example, two housing elements may be used to house a total of four batteries or other power sources. In this embodiment, two batteries would be installed at all times, and ejection mechanism 34 prevents removal of more than two batteries or power sources at a time from a given housing 22.

The term battery as used herein includes multiple stacked batteries fit within a single battery housing. First power source 26, for example, can include multiple stacked cells placed in first housing part 45. The cells may be stacked vertically or horizontally. Second and third projection members 64 and 66 may be increased in length or height to assure that they contact the rear surface of all the stacked cells.

In an example embodiment, compression spring-based elements (not shown), placed behind first and second rods 42, 40 may be used together with or as an alternative to spring-based elements 44, 46. The rear wall of the housing 22 (not shown in Figure 7 for clarity) may provide a surface against which the compression spring-based elements could bear.

In an example embodiment, bearings (not shown) or a low friction surface material may be provided on an inside bottom surface of housing 22 to facilitate forward movement and removal of first and second power sources 26, 28.

In an example embodiment, parts of ejection mechanism 34 may be replaced with an electro-mechanical servo and switch system. A first switch inside housing 22 may be used to detect the presence of first power source 26 and a second switch inside housing 22 may be used to detect the presence of second power source 28. When control unit 16 in communication with the switches determines that only the first switch is triggered, i.e., only second power source 28 is present in housing 22, it directs the servo to lock second power source 28 in housing 22, for example, by rotating knob 30 such that it lies in front of power source 28 but not first power source 26. Similarly, when only the second switch is triggered, the control unit 16 directs the servo to lock first power source 26 in housing 22 by rotating the knob 30 in the opposite direction such that it lies in front of first power source 26. The servo may also be connected to any other type of locking mechanism. As indicated above, control unit 16 may impose additional conditions which must be met before it allows knob 30 to open.

In an example embodiment, solenoids can be utilized to fix movement of knob 30. Similar to the embodiment illustrated in Figure 5, solenoids (not shown) can be directly or indirectly connected to rods of which the forward end 68 is extendible into recess 70 of knob 30. The solenoids can be located in the front or the back of the power-source housing 22, and the rod length can be adjusted accordingly. When control unit 16 determines that either side of the power-source housing 22 should be locked based on the status of the power sources, the control unit 16 controls a solenoid to engage the forward end 68 of the appropriate rod with recess 70 in the knob, effectively fixing knob 30 and thus locking the opening of the power-source housing 22. This embodiment can be operated with spring-based elements that put forward pressure on the power source, as illustrated in Figure 2D or in Figure 6.

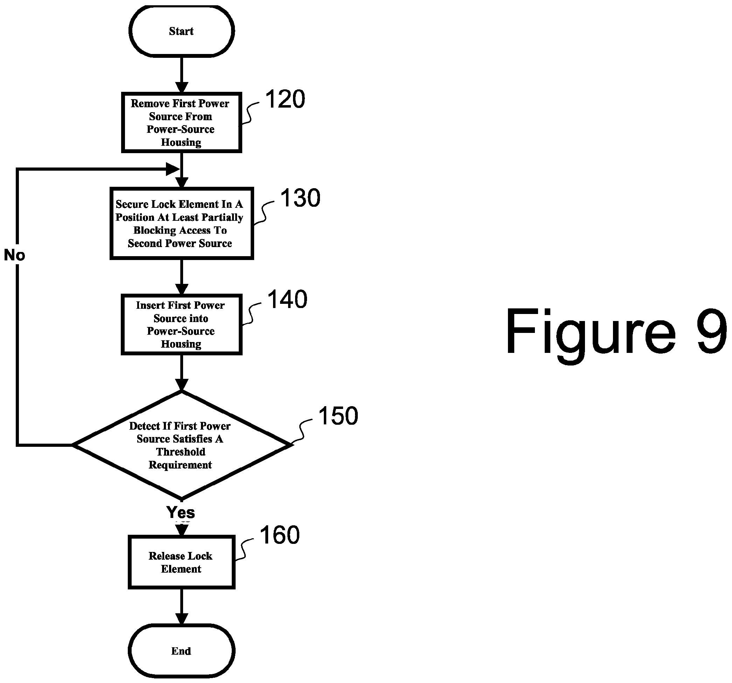

Figure 9 illustrates a method according to an example embodiment of the present invention for fixing the lock element or knob 30 over the power source that the IABP 10 determines should not be removed. In Step 120, a user removes a first power source. For removal of the first power source, the locking element is in a position that prevents access to the second power source. In Step 130, the locking element is fixed in the position that prevents access to the second power source. In Step 140, a user introduces a first power source into the power-source housing. In Step 150, the control unit determines whether the first power source satisfies a threshold requirement. A threshold requirement can be determined based on detectable characteristics of the power sources such as any combination of voltage, current, temperature, expected battery life remaining, manufacturer, etc. If the first power source satisfies the threshold requirement, then, in Step 160, the control unit defixes or releases the lock element so that user can move the lock element. If not, the control unit maintains the lock secured.

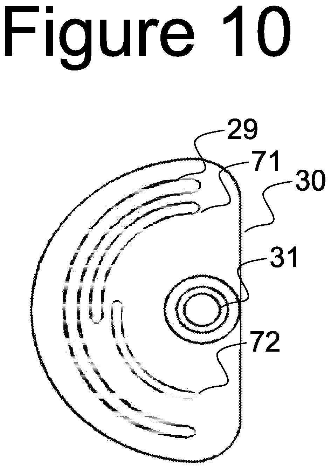

In addition to or in place of hole recesses 70 used in the embodiment shown in Figure 4B, knob 30 can include grooves to restrict the direction of rotation. Figure 10 illustrates an exemplary knob containing partial grooves 71, 72. These recess partial grooves 71, 72 are used in connection with a solenoid-controlled rod that enters the grooves based on the IABP's detection of the power source states or operating characteristics. Partial recess groove 71 restricts rotation of knob 30 from the position in Figure 2A blocking both first and second power sources 26, 28 to the position of Figure 2B allowing access to one of power sources 26, 28. In a similar manner, partial recess groove 71 restricts rotation from the position in Figure 2A blocking both first and second power sources 26, 28 to the position of Figure 2C allowing access to one of power sources 26, 28. Recess groove 29 is preferably included, but optional, to facilitate smooth rotation. Figure 10 also illustrates recess groove 29 and partial recess grooves 71, 72 as having different widths. Likewise, these grooves may be adapted to have similar and/or different depths and widths to each other depending on the system they are used in. As shown in Figure 4B and described above, recess holes 70 (not shown in Figure 10) can optionally be used for locking or snapping into a position.

Sensing of the existence and characteristics of particular power sources can be checked in multiple ways. Switches can detect and limit the amount of power going through them. A system bus controller (not shown) may be used to detect and store which power sources are present, the amount of current drawn from the power sources, the amount of voltage in the power sources, the temperature of the power sources, etc. In addition, the system bus controller may detect information that can be stored in the power sources themselves. For example, the power sources may have memory in the form of circuitry or software that contains (i) error codes about states of the power source, (ii) the amount of remaining expected life in a battery or (iii) a temperature inside a power supply system.

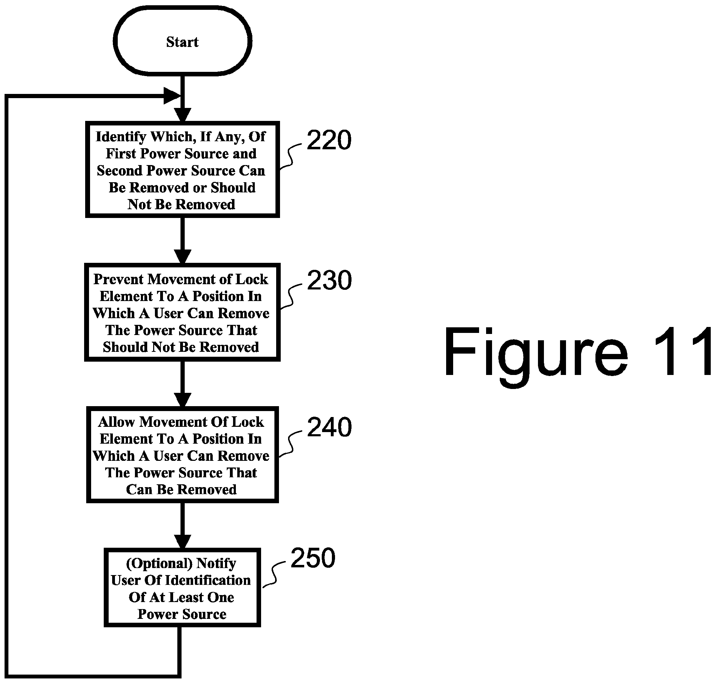

Figure 11 illustrates an example method that prevents movement of a locking element, such as knob 30, to a position that allows access to a power source that the IABP 10 determines should not be removed. In Step 220, the control unit identifies based on a detection of power source characteristics whether a power source that is present should not be removed and whether a power source is present that can be removed. In Step 230, the control unit prevents movement of the lock element to a position that allows access to the power source identified that should not be removed. In Step 240, the control unit allows movement of the lock element to a position that allows access to the power source identified as can be removed. The control unit can optionally notify the user of this identification through status lights 23 on the power source, status lights on the power source compartment, IABP 10 or locking element (not shown) or through the user input interface and display 18.

Although IABP 10 is described above, the exemplary embodiments described above can be used in other similar systems where maintaining power is important, especially medical devices and systems used in a hospital, such as a heart-lung machine.
Those skilled in the art can appreciate from the foregoing description that the present invention can be implemented in a variety of forms. Therefore, while the embodiments of this invention have been described in connection with particular examples thereof, the true scope of the embodiments of the invention should not be so limited since other modifications and variations will become apparent to the skilled practitioner upon a study of the drawings and specification. Such modifications and variations are considered to be within the purview and scope of the appended claims and their equivalents.

## Claims

1. An intra-aortic balloon pump system comprising:
a pump (14) for inflating and deflating a balloon of an intra-aortic balloon catheter; a first power source (26) for supplying power to the pump;
a second power source (28) for supplying power to the pump; and
a power supply system (12);
the power supply system (12) being **characterized by**:
a first housing part (45) configured to removably receive the first power source (26) through a first opening (41) of the first housing part (45);
a second housing part (47) configured to removably receive the second power source (28) through a second opening (43) of the second housing part (47); and
a lock element movably positioned with respect to the first and second housing parts (45, 47) to prevent simultaneous removal of the first and second power sources (26, 28) from the first and second housing parts (45, 47), wherein the lock element is configured to secure the first power source (26) within the first housing part (45) when the second power source (28) is removed from the second housing part (47) and wherein the lock element is configured to secure the second power source (28) within the second housing part (47) when the first power source (26) is removed from the first housing part (45), and wherein the lock element is configured to secure the first power source (26) within the first housing part (45) and to secure the second power source (28) within the second housing part (47) when both the first power source (26) and the second power source (28) are disposed within the first housing part (45) and the second housing part (47), respectively.

2. A method for using the power supply system (12) of the intra-aortic balloon pump system according to claim 1, wherein the method comprises the steps of:
movably positioning the lock element relative to the first and second housing parts (45, 47) to secure the first power source (26) within the first housing part (45) when the second power source (28) is removed from the second housing part (47); and
movably positioning the lock element relative to the first and second housing parts (45, 47) to secure the second power source (28) within the second housing part (47) when the first power source (26) is removed from the first housing part (45) so as to prevent simultaneous removal of the first and second power sources (26, 28) from the first and second housing parts (45, 47).

3. The method of claim 2, further comprising the steps of movably positioning the lock element relative to the first and second housing parts (45, 47) to secure the first power source (26) within the first housing part (45) and simultaneously securing the second power source (28) within the second housing part (47).

4. The method of any one of claims 2-3, further comprising the steps of moving the lock element to a first position at least partially blocking the first opening (41) when the first power source (26) is contained within the first housing part (45) while permitting unobstructed access to the second opening (43) and removing the second power source (28) from the second housing part (47).

5. The intra-aortic balloon pump system of claim 1, wherein the first and second power sources (26, 28) are different types of power sources.

6. The intra-aortic balloon pump system of claim 5, wherein the first power source (26) is a battery and the second power source (28) is an AC power-supply module.

7. The intra-aortic balloon pump system of claim 1, wherein the lock element comprises a rotatable knob (30).

8. The intra-aortic balloon pump system of claim 7, wherein the rotatable knob (30) has a perimeter comprising an arcuate portion and a linear portion.

9. The intra-aortic balloon pump system of claim 8, wherein the rotatable knob (30) is dimensioned to secure the first power source (26) within the first housing part (45) and to secure the second power source (28) within the second housing part (47) when both the first power source (26) and the second power source (28) are disposed within the first housing part (45) and the second housing part (47), respectively, wherein the rotatable knob (30) secures the first power source (26) and the second power source (28) by partially blocking the first opening (41) and the second opening (43).

## Patentansprüche

1. Intraaortisches Ballonpumpensystem, das Folgendes umfasst:
eine Pumpe (14) zum Aufblasen und Entleeren eines Ballons eines intraaortischen Ballonkatheters; eine erste Stromquelle (26) zum Versorgen der Pumpe mit Strom;
eine zweite Stromquelle (28) zum Versorgen der Pumpe mit Strom; und
ein Stromversorgungssystem (12);
wobei das Stromversorgungssystem (12) durch Folgendes gekennzeichnet ist:
ein erstes Gehäuseteil (45), das dazu konfiguriert ist, die erste Stromquelle (26) durch eine erste Öffnung (41) des ersten Gehäuseteils (45) entfernbar aufzunehmen;
ein zweites Gehäuseteil (47), das dazu konfiguriert ist, die zweite Stromquelle (28) durch eine zweite Öffnung (43) des zweiten Gehäuseteils (47) entfernbar aufzunehmen; und
ein Verriegelungselement, das in Bezug auf das erste und das zweite Gehäuseteil (45, 47) bewegbar positioniert ist, um gleichzeitiges Entfernen der ersten und zweiten Stromquellen (26, 28) aus dem ersten und zweiten Gehäuseteil (45, 47) zu verhindern, wobei das Verriegelungselement dazu konfiguriert ist, die erste Stromquelle (26) innerhalb des ersten Gehäuseteils (45) zu sichern, wenn die zweite Stromquelle (28) aus dem zweiten Gehäuseteil (47) entfernt wird, und wobei das Verriegelungselement dazu konfiguriert ist, die zweite Stromquelle (28) innerhalb des zweiten Gehäuseteils (47) zu sichern, wenn die erste Stromquelle (26) aus dem ersten Gehäuseteil (45) entfernt wird, und wobei das Verriegelungselement dazu konfiguriert ist, die erste Stromquelle (26) innerhalb des ersten Gehäuseteils (45) zu sichern und die zweite Stromquelle (28) innerhalb des zweiten Gehäuseteils (47) zu sichern, wenn sowohl die erste Stromquelle (26) als auch die zweite Stromquelle (28) innerhalb des ersten Gehäuseteils (45) bzw. des zweiten Gehäuseteils (47) angeordnet sind.

2. Verfahren zum Verwenden des Stromversorgungssystems (12) des intraaortischen Ballonpumpensystems nach Anspruch 1, wobei das Verfahren die folgenden Schritte umfasst:
bewegliches Positionieren des Verriegelungselements relativ zu dem ersten und zweiten Gehäuseteil (45, 47), um die erste Stromquelle (26) innerhalb des ersten Gehäuseteils (45) zu sichern, wenn die zweite Stromquelle (28) aus dem zweiten Gehäuseteil (47) entfernt ist; und
bewegliches Positionieren des Verriegelungselements relativ zu dem ersten und zweiten Gehäuseteil (45, 47), um die zweite Stromquelle (28) innerhalb des zweiten Gehäuseteils (47) zu sichern, wenn die erste Stromquelle (26) aus dem ersten Gehäuseteil (45) entfernt ist, um gleichzeitiges Entfernen der ersten und zweiten Stromquelle (26, 28) aus dem ersten und zweiten Gehäuseteil (45, 47) zu verhindern.

3. Verfahren nach Anspruch 2, das ferner die Schritte des beweglichen Positionierens des Verriegelungselements relativ zu dem ersten und zweiten Gehäuseteil (45, 47) umfasst, um die erste Stromquelle (26) innerhalb des ersten Gehäuseteils (45) zu sichern und gleichzeitig die zweite Stromquelle (28) innerhalb des zweiten Gehäuseteils (47) zu sichern.

4. Verfahren nach einem der Ansprüche 2-3, das ferner die Schritte des Bewegens des Verriegelungselements in eine erste Position, welche die erste Öffnung (41) mindestens teilweise blockiert, umfasst, wenn die erste Stromquelle (26) innerhalb des ersten Gehäuseteils (45) enthalten ist, während ungehinderter Zugang zu der zweiten Öffnung (43) und Entfernen der zweiten Stromquelle (28) aus dem zweiten Gehäuseteil (47) ermöglicht werden.

5. Intraaortisches Ballonpumpensystem nach Anspruch 1, wobei die erste und zweite Stromquelle (26, 28) unterschiedliche Arten von Stromquellen sind.

6. Intraaortisches Ballonpumpensystem nach Anspruch 5, wobei die erste Stromquelle (26) eine Batterie ist und die zweite Stromquelle (28) ein Wechselstromversorgungsmodul ist.

7. Intraaortisches Ballonpumpensystem nach Anspruch 1, wobei das Verriegelungselement einen drehbaren Knauf (30) umfasst.

8. Intraaortisches Ballonpumpensystem nach Anspruch 7, wobei der drehbare Knauf (30) einen Umfang aufweist, der einen bogenförmigen Abschnitt und einen linearen Abschnitt aufweist.

9. Intraaortisches Ballonpumpensystem nach Anspruch 8, wobei der drehbare Knauf (30) dazu bemessen ist, die erste Stromquelle (26) innerhalb des ersten Gehäuseteils (45) zu sichern und die zweite Stromquelle (28) innerhalb des zweiten Gehäuseteils (47) zu sichern, wenn sowohl die erste Stromquelle (26) als auch die zweite Stromquelle (28) innerhalb des ersten Gehäuseteils (45) und des zweiten Gehäuseteils (47) angeordnet sind, wobei der drehbare Knauf (30) die erste Stromquelle (26) und die zweite Stromquelle (28) durch teilweises Blockieren der ersten Öffnung (41) und der zweiten Öffnung (43) sichert.

## Revendications

1. Système de pompe à ballonnet intra-aortique comprenant :
une pompe (14) pour gonfler et dégonfler un ballonnet d'un cathéter à ballonnet intra-aortique ; une première source d'électricité (26) pour fournir de l'électricité à la pompe ;
une seconde source d'électricité (28) pour fournir de l'électricité à la pompe ; et
un système d'alimentation électrique (12) ;
le système d'alimentation électrique (12) étant **caractérisé par** :
une première partie de logement (45) configurée pour recevoir de façon amovible la première source d'électricité (26) au travers d'une première ouverture (41) de la première partie de logement (45) ;
une seconde partie de logement (47) configurée pour recevoir de façon amovible la seconde source d'électricité (28) au travers d'une seconde ouverture (43) de la seconde partie de logement (47) ; et
un élément de verrouillage positionné de façon mobile par rapport aux première et seconde parties de logement (45, 47) pour empêcher un retrait simultané des première et seconde sources d'électricité (26, 28) des première et seconde parties de logement (45, 47), dans lequel l'élément de verrouillage est configuré pour fixer la première source d'électricité (26) dans la première partie de logement (45) lorsque la seconde source d'électricité (28) est retirée de la seconde partie de logement (47) et dans lequel l'élément de verrouillage est configuré pour fixer la seconde source d'électricité (28) dans la seconde partie de logement (47) lorsque la première source d'électricité (26) est retirée de la première partie de logement (45), et dans lequel l'élément de verrouillage est configuré pour fixer la première source d'électricité (26) dans la première partie de logement (45) et pour fixer la seconde source d'électricité (28) dans la seconde partie de logement (47) lorsqu'à la fois la première source d'électricité (26) et la seconde source d'électricité (28) sont disposées dans la première partie de logement (45) et la seconde partie de logement (47), respectivement.

2. Procédé pour l'utilisation du système d'alimentation électrique (12) du système de pompe à ballonnet intra-aortique selon la revendication 1, dans lequel le procédé comprend les étapes consistant à :
positionner de façon mobile l'élément de verrouillage par rapport aux première et seconde parties de logement (45, 47) pour fixer la première source d'électricité (26) dans la première partie de logement (45) lorsque la seconde source d'électricité (28) est retirée de la seconde partie de logement (47) ; et
positionner de façon mobile l'élément de verrouillage par rapport aux première et seconde parties de logement (45, 47) pour fixer la seconde source d'électricité (28) dans la seconde partie de logement (47) lorsque la première source d'électricité (26) est retirée de la première partie de logement (45) de façon à empêcher un retrait simultané des première et seconde sources d'électricité (26, 28) des première et seconde parties de logement (45, 47).

3. Procédé selon la revendication 2, comprenant en outre les étapes consistant à positionner de façon mobile l'élément de verrouillage par rapport aux première et seconde parties de logement (45, 47) pour fixer la première source d'électricité (26) dans la première partie de logement (45) et fixer simultanément la seconde source d'électricité (28) dans la seconde partie de logement (47).

4. Procédé selon l'une quelconque des revendications 2 à 3, comprenant en outre les étapes consistant à déplacer l'élément de verrouillage d'une première position bloquant au moins partiellement la première ouverture (41) lorsque la première source d'électricité (26) est contenue dans la première partie de logement (45) tout en permettant un accès non obstrué à la seconde ouverture (43) et à retirer la seconde source d'électricité (28) de la seconde partie de logement (47).

5. Système de pompe à ballonnet intra-aortique selon la revendication 1, dans lequel les première et seconde sources d'électricité (26, 28) sont des sources d'électricité de types différents.

6. Système de pompe à ballonnet intra-aortique selon la revendication 5, dans lequel la première source d'électricité (26) est une batterie et la seconde source d'électricité (28) est un module de fourniture de courant alternatif.

7. Système de pompe à ballonnet intra-aortique selon la revendication 1, dans lequel l'élément de verrouillage comprend un bouton rotatif (30).

8. Système de pompe à ballonnet intra-aortique selon la revendication 7, dans lequel le bouton rotatif (30) a un périmètre comprenant une partie arquée et une partie linéaire.

9. Système de pompe à ballonnet intra-aortique selon la revendication 8, dans lequel le bouton rotatif (30) est dimensionné pour fixer la première source d'électricité (26) dans la première partie de logement (45) et pour fixer la seconde source d'électricité (28) dans la seconde partie de logement (47) lorsqu'à la fois la première source d'électricité (26) et la seconde source d'électricité (28) sont disposées dans la première partie de logement (45) et la seconde partie de logement (47), respectivement, dans lequel le bouton rotatif (30) fixe la première source d'électricité (26) et la seconde source d'électricité (28) en bloquant partiellement la première ouverture (41) et la seconde ouverture (43).
